# EUROPÄISCHE PATENTSCHRIFT

(11) **EP 4 008 364 B1**
(45) Veröffentlichungstag und Bekanntmachung des Hinweises auf die Patenterteilung: **14.08.2024**
(21) Anmeldenummer: 21208344.8
(22) Anmeldetag: 15.11.2021
(51) Int. Cl.: A61L 2/18, A61L 2/24, B08B 9/42, B29C 49/42

(54) **VORRICHTUNG UND VERFAHREN ZUM STERILISIEREN VON KUNSTSTOFFVORFORMLINGEN**
APPARATUS AND METHOD FOR STERILISING PLASTIC PRE-FORMS
DISPOSITIF ET PROCÉDÉ DE STÉRILISATION DE PRÉFORMES EN MATIÈRE PLASTIQUE

(30) Priorität: 04.12.2020 DE 102020132324
(43) Veröffentlichungstag der Anmeldung: 08.06.2022
(73) Patentinhaber: Krones AG, 93073 Neutraubling (DE)
(72) Erfinder: Suppes, Waldemar, 93073 Neutraubling (DE); Geltinger, Florian, 93073 Neutraubling (DE)
(74) Vertreter: Hannke Bittner & Partner mbB Regensburg

(56) Entgegenhaltungen:
- DE-A1- 102008 038 143
- DE-A1- 102010 049 385
- DE-A1- 102011 107 772
- DE-A1- 102012 112 158
- DE-A1- 102012 112 803
- DE-A1- 102018 124 287

## Beschreibung

Die vorliegende Erfindung bezieht sich auf eine Vorrichtung und ein Verfahren zum Sterilisieren von Behältnissen, insbesondere von Kunststoffbehältnissen und insbesondere von Kunststoffvorformlingen. Derartige Kunststoffvorformlinge werden im Stand der Technik eingesetzt, um erwärmt und später zu Kunststoffbehältnissen wie beispielsweise Kunststoffflaschen umgeformt zu werden.

Dabei sind aus dem Stand der Technik unterschiedliche Vorgehensweisen zum Sterilisieren dieser Kunststoffvorformlinge bekannt. So ist es beispielsweise bekannt, die Kunststoffvorformlinge mittels Strahlung wie beispielsweise Elektronenstrahlung oder Röntgenstrahlung oder UV-Strahlung zu sterilisieren. Bei einem anderen Konzept werden die Kunststoffvorformlinge mit einer sterilisierenden Substanz wie beispielsweise Peressigsäure oder Wasserstoffperoxid behandelt.

Zu diesem Zweck ist es im Stand der Technik bekannt, dass die Behältnisse auf einen Transportstern verbracht werden und an diesem Transportstern während ihres Transportes mit einem Sterilisationsmedium beaufschlagt werden. Im Stand der Technik finden dabei beispielsweise mehrere dieser Transportsterne hintereinander Anwendung. Üblicherweise ist dabei ein erster Transportstern vorgesehen, auf dem die Kunststoffvorformlinge behandelt werden an diesen schließt sich ein weiterer Transportstern an, der die Kunststoffvorformlinge nur transportiert und an diesem wiederum ein Transportstern, an dem sowohl ein Transport als auch ein Beaufschlagen der Kunststoffvorformlinge erfolgt. Diese Vorgehensweisen haben sich technisch bewährt, erfordern jedoch relativ viel Platz innerhalb einer Behältnisbehandlungsanlage.

Aus der DE 10 2008 038 143 A1 ist beispielsweise eine Vorrichtung und ein Verfahren zum Herstellen von Kunststoffbehältnissen bekannt, wobei mit einer Sterilisationseinrichtung eine Sterilisation nach dem Ende des Heizvorgangs der Behältnisse und vor dem Ende des Umformungsvorgangs stattfindet. Weiterhin ist aus der DE 10 2018 124 287 A1 ein Verfahren und eine Vorrichtung zum Sterilisieren von Preforms mittels Sterilisationsdampf bekannt, wobei Teile oder Komponenten erwärmt werden und/oder ein Teil des Sterilisationsdampfes durch Kondensation an Kühlelementen verflüssigt wird, um eine Kondensation des Dampfes zu vermeiden.

Auch aus der DE 10 2012 112 803 A1, der DE 10 2012 112 158 A1 und der DE 10 2011 107 772 A1 sind bereits Vorrichtungen und Verfahren zum Sterilisieren von Kunststoffvorformlingen bekannt.

Der vorliegenden Erfindung liegt daher die Aufgabe zu Grunde, den Platz und/oder den Bauraum für solche Anlagen zu reduzieren. Auch soll eine Möglichkeit geschaffen werden, derartige Behandlungen effizienter zu gestalten. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Behandeln von Kunststoffvorformlingen weist eine Erwärmungseinrichtung zum Erwärmen der Kunststoffvorformlinge auf, wobei die Erwärmungseinrichtung eine Transporteinrichtung zum Transportieren der Kunststoffvorformlinge aufweist, sowie wenigstens eine Heizeinrichtung zum Erwärmen der Kunststoffvorformlinge. Weiterhin weist die Vorrichtung eine in einer Transportrichtung der Kunststoffvorformlinge der Erwärmungseinrichtung nachgeordnete Umformungseinrichtung zum Umformen der Kunststoffvorformlinge zur Kunststoffbehältnissen auf, sowie eine Sterilisationseinrichtung zum Sterilisieren der Kunststoffvorformlinge.

Erfindungsgemäß weist die Sterilisationseinrichtung wenigstens eine erste Sterilisationseinheit auf, welche einen drehbaren Transportträger und eine Vielzahl von an diesem Transportträger angeordneten ersten Halteeinrichtungen zum Halten der Kunststoffvorformlinge aufweist sowie auch eine Vielzahl von ersten Beaufschlagungseinrichtungen zum Beaufschlagen der Kunststoffvorformlinge mit einem fließfähigem Medium.

Weiterhin weist die Sterilisationseinrichtung eine zweite Sterilisationseinheit auf, welche sich in der Transportrichtung der Kunststoffvorformlinge an die erste Sterilisationseinheit anschließt und welche einen drehbaren Transportträger und eine Vielzahl von an diesem Transportträger angeordneten zweiten Halteeinrichtungen zum Halten der Kunststoffvorformlinge aufweist. Daneben weist diese zweite Sterilisationseinheit eine Vielzahl von zweiten Beaufschlagungseinrichtungen zum Beaufschlagen der Kunststoffvorformlinge mit einem fließfähigen Medium auf, wobei die Kunststoffvorformlinge unmittelbar von der ersten Sterilisationseinheit an die zweite Sterilisationseinheit übergebbar sind.

Es wird daher im Rahmen der Erfindung vorgeschlagen, dass die Sterilisationseinrichtung derart aufgebaut ist, dass die Kunststoffvorformlinge unmittelbar von der ersten Sterilisationseinheit an die zweite Sterilisationseinheit übergebbar sind. Dies bedeutet insbesondere, dass die Kunststoffvorformlinge beispielsweise von einer Halteeinrichtung der ersten Sterilisationseinheit unmittelbar an eine Halteeinrichtung der zweiten Sterilisationseinheit übergeben werden, wohingegen im Stad der Technik zunächst die Kunststoffvorformlinge von der ersten Sterilisationseinheit an eine Transporteinrichtung übergeben werden und von dieser wiederum an die zweite Sterilisationseinheit.

Bevorzugt handelt es sich bei dem fließfähigen Medium, mit welchem die ersten Beaufschlagungseinrichtungen die Kunststoffvorformlinge beaufschlagen, um ein Sterilisations- und/oder ein Reinigungsmedium. Insbesondere handelt es sich bei dem fließfähigen Medium um ein flüssiges Medium und insbesondere um eine Luftmischung mit flüssigem H2O2 bzw. um H2O2-Prozessgas. Es wäre jedoch auch Peressigsäure verwendbar.

Bevorzugt handelt es sich bei dem fließfähigen Medium, mit welchem die zweiten Beaufschlagungseinrichtungen die Kunststoffvorformlinge beaufschlagen, um ein Sterilisations- und/oder ein Reinigungsmedium. Insbesondere handelt es sich bei dem fließfähigen Medium um ein flüssiges Medium und insbesondere um Wasserstoffperoxid oder Peressigsäure.

Im Rahmen der Erfindung wird Bezug genommen auf Sterilisationseinheiten. Es wird jedoch darauf hingewiesen, dass es sich neben oder anstelle der Sterilisationseinheiten auch um Reinigungseinheiten handeln könnte, welche die Kunststoffvorformlinge mit einem Reinigungsmedium beaufschlagen. Bevorzugt handelt es sich jedoch um Sterilisationseinheiten. Auch bei der Sterilisationseinrichtung in ihrer Gesamtheit könnte es sich um eine Reinigungseinrichtung handeln, bevorzugt handelt es sich jedoch um eine Sterilisationseinrichtung.

Dabei ist es möglich, dass die erste und die zweite Sterilisationseinheit die Behältnisse mit dem gleichen Sterilisationsmedium und/oder Reinigungsmedium beaufschlagen. Es wäre jedoch auch möglich, dass die erste und die zweite Sterilisationseinheit die Kunststoffvorformlinge mit dem gleichen Sterilisationsmedium und/oder Reinigungsmedium beaufschlagen.

Vorteilhaft handelt es sich bei der Erwärmungseinrichtung um eine Infrarot - Erwärmungseinrichtung und insbesondere einen Infrarot - Ofen. Bevorzugt sind die Heizelemente bzw. Heizeinrichtungen dieser Erwärmungseinrichtung stationär angeordnet und die Kunststoffvorformlinge werden an diesen Heizeinrichtungen vorbei transportiert. Es könnte sich bei der Erwärmungseinrichtung jedoch auch um einen Mikrowellenofen handeln, der die Kunststoffvorformling durch Beaufschlagung mit Mikrowellenstrahlung erwärmt.

Weiterhin weist die Erwärmungseinrichtung eine Dreheinrichtung zum Drehen der Kunststoffvorformlinge bezüglich der Längsrichtungen auf. Auf diese Weise wird eine gleichmäßigere Erwärmung erreicht.

Bei einer weiteren Vorteilhaften Ausführungsform handelt es sich bei der Umformungseinrichtung um eine Blasformmaschine und insbesondere um eine Streckblasmaschine. Bevorzugt weist diese Umformungseinrichtung eine Vielzahl von Umformungsstationen auf, in welche die Kunststoffvorformlinge eingegeben und dort mittels eines unter Druck stehenden Mediums, insbesondere mit Hilfe von Blasluft aber gegebenenfalls auch mit einem entsprechenden Getränk zu den Behältnissen umgeformt werden.

Bei einer weiteren bevorzugten Ausführungsform weist die Umformungseinrichtung eine Vielzahl von stangenartigen Körpern (sogenannte Reckstangen) auf, welche in die Kunststoffvorformlinge einführbar sind, um diese in ihrer Längsrichtung zu dehnen. Bevorzugt weisen die Umformungsstationen der Umformungseinrichtungen weiterhin so genannte Blasdüsen auf, welche bevorzugt an die Mündungen der Kunststoffvorformlinge anlegbar sind, um diese so zu expandieren.

Bei den oben erwähnten Halteeinrichtungen zum Halten der Kunststoffvorformlinge handelt es sich insbesondere um Greifklammern, welche die Kunststoffvorformlinge in einem vorgegebenen Bereich, beispielsweise unterhalb oder oberhalb ihres so genannten Tragrings halten.

Bei einer bevorzugten Ausführungsform sind die Kunststoffvorformlinge unmittelbar von einer Halteeinrichtung der ersten Sterilisationseinheit an eine Halteeinrichtung der zweiten Sterilisationseinheit übergebbar.

Unter der Sterilisationseinrichtung wird im Rahmen der vorliegenden Anmeldung die gesamte Einrichtung verstanden, die zum Sterilisieren und/oder Reinigen der Behältnisse dient. Die Sterilisationseinheiten sind die einzelnen Aggregate dieser Einrichtung, also insbesondere Transportsterne mit daran angeordneten Halteeinrichtungen und Beaufschlagungseinrichtungen.

Bei einer bevorzugten Ausführungsform sind die die Sterilisationseinheiten als Behandlungssterne ausgeführt, d. h. sie weisen bevorzugt drehbare Träger auf, an denen die Halteeinrichtungen und/oder Beaufschlagungseinrichtungen angeordnet sind. Dabei ist es möglich, dass diese Sterilisationseinheiten mit speziellen Düsen ausgestattet sind, welche das Innere der Kunststoffvorformlinge mit einem Sterilisationsmittel und Insbesondere mit Wasserstoffperoxid befüllen und/oder auffüllen.

Bei einer weiteren vorteilhaften Ausführungsform ist die Sterilisationseinrichtung in der Transportrichtung der Kunststoffvorformlinge zwischen der Erwärmungseinrichtung und der Umformungseinrichtung angeordnet. Dies bedeutet, dass die Kunststoffvorformlinge zunächst in einem Ofen erwärmt werden und anschließend in diesem erwärmten Zustand sterilisiert und anschließend auch zu den Kunststoffbehältnissen umgeformt, beispielsweise geblasen werden.

Es wäre doch auch denkbar, dass die Sterilisationseinrichtung vor der Erwärmungseinrichtung angeordnet ist das heißt die Kunststoffvorformlinge zunächst sterilisiert und anschließend erwärmt werden.

Die vorliegende Erfindung ist weiterhin auf eine Sterilisationseinrichtung zum Sterilisieren von Kunststoffbehältnissen und insbesondere von Kunststoffvorformlingen gerichtet, wobei die Sterilisationseinrichtung wenigstens eine erste Sterilisationseinheit aufweist, welche einen drehbaren Transportträger und eine Vielzahl an diesem Transportträger angeordneten ersten Halteeinrichtungen zum Halten der Kunststoffvorformlinge sowie eine Vielzahl von ersten Beaufschlagungseinrichtungen zum Beaufschlagen der Kunststoffvorformlinge mit einem fließfähigem Sterilisationsmedium aufweist und die Sterilisationseinrichtung eine zweite Sterilisationseinheit aufweist, welche sich einer Transportrichtung der Kunststoffvorformlinge an die erste Sterilisationseinheit anschließt und welche einen drehbaren Transportträger und eine Vielzahl von an diesem Transportträger angeordneten zweiten Halteeinrichtungen zum Halten der Kunststoffvorformlinge aufweist sowie eine Vielzahl von zweiten Beaufschlagungseinrichtungen zum Beaufschlagen der Kunststoffvorformlinge mit einem fließfähigem Sterilisationsmedium.

Erfindungsgemäß sind die Kunststoffvorformlinge unmittelbar von der ersten Sterilisationseinheit an die zweite Sterilisationseinheit übergebbar.

Weiterhin ist es möglich, dass ein erster drehbarer Träger vorgesehen ist, an dem die Halteeinrichtungen angeordnet sind, sowie ein zweiter drehbarer Träger, an dem die Beaufschlagungseinrichtungen vorgesehen sind. Bevorzugt sind diese beiden drehbaren Träger um die gleiche Rotationsachse drehbar. Bevorzugt sind diese beiden Transportträger auch parallel zueinander.

Bei einer weiteren bevorzugten Ausführungsform ist jeder Beaufschlagungseinrichtung eine Zuführleitung zugeordnet, welche dieser Beaufschlagungseinrichtung das Sterilisationsmedium zuführt. Bei einer weiteren vorteilhaften Ausführungsform weist die Vorrichtung eine Verteileinrichtung auf, welche ein Sterilisationsmedium auf die einzelnen Zuführleitungen und/oder die einzelnen Beaufschlagungseinrichtungen aufteilt.

Bei einer weiteren vorteilhaften Ausführungsform ist die Verteileinrichtung als Drehverteiler ausgeführt, welcher ausgehend aus einem Reservoir das Sterilisationsmedium an die einzelnen Beaufschlagungseinrichtungen verteilt.

Bei einer bevorzugten Ausführungsform ist jeder ersten Halteeinrichtung genau eine erste Beaufschlagungseinrichtung zugeordnet und/oder jeder zweiten Halteeinrichtung ist genau eine zweite Beaufschlagungseinrichtung zugeordnet. Bevorzugt sind dabei die Halteeinrichtungen unterhalb der Beaufschlagungseinrichtungen angeordnet.

Bei einer weiteren bevorzugten Ausführungsform laufen die ersten Beaufschlagungseinrichtungen damit um die gleiche Drehachse um wie die ersten Halteeinrichtungen. Bevorzugt laufen auch die zweiten Beaufschlagungseinrichtungen jeweils um die gleiche Drehachse um wie die zweiten Beaufschlagungseinrichtungen.

Bevorzugt weist die erste und oder die zweite Sterilisiereinheit wenigstens 10, bevorzugt wenigstens 20 und bevorzugt wenigstens 30 Beaufschlagungseinrichtungen und/oder Halteeinrichtungen auf. Bei einer weiteren bevorzugten Ausführungsform weist die erste und oder die zweite Sterilisationseinheit höchstens 100, bevorzugt höchstens 90 und bevorzugt höchstens 80 Beaufschlagungseinrichtungen und/oder Halteeinrichtungen auf.

Bei einer weiteren bevorzugten Ausführungsform sind die ersten Beaufschlagungseinrichtungen und/oder die zweiten Beaufschlagungseinrichtungen an einem drehbaren Träger angeordnet. Dabei kann es sich um den gleichen drehbaren Träger halten, an dem auch die Halteeinrichtungen angeordnet sind. Bevorzugt sind jedoch die Beaufschlagungseinrichtungen in einem anderen drehbaren Träger angeordnet als die Halteeinrichtungen.

Bei einer weiteren bevorzugten Ausführungsform weist die Sterilisationseinrichtung eine dritte Sterilisationseinheit auf, welche einen drehbaren Transportträger und eine Vielzahl von an diesem Transportträger angeordnetem Halteeinrichtungen zum Halten der Kunststoffvorformlinge sowie eine Vielzahl von Beaufschlagungseinrichtungen zum Beaufschlagen der Kunststoffvorformlinge mit einem fließfähigem Sterilisationsmedium aufweist.

Bevorzugt schließt sich diese dritte Sterilisationseinheit an die zweite Sterilisationseinheit an. Besonders bevorzugt sind die Kunststoffvorformlinge unmittelbar von der zweiten Sterilisationseinheit an die dritte Sterilisationseinheit übergebbar und/oder genau genommen von einer Halteeinrichtung der zweiten Sterilisationseinheit an eine Halteeinrichtung der dritten Sterilisationseinheit.

Erfindungsgemäß sind die ersten Beaufschlagungseinrichtungen in einer ersten Position bezüglich einer Längsrichtung der Kunststoffvorformlinge angeordnet und die zweiten Beaufschlagungseinrichtungen sind in einer zweiten Position bezüglich der Längsrichtung der Kunststoffvorformlinge angeordnet und bevorzugt unterscheiden sich die ersten und die zweiten Positionen bzw. sind unterschiedlich.

Bevorzugt sind daher die ersten und die zweiten Beaufschlagungseinrichtungen und/oder Bestandteile der ersten und zweiten Beaufschlagungseinrichtungen auf unterschiedlichen Höhen und Positionen angeordnet. Auf diese Weise können die ersten und die zweiten Beaufschlagungseinrichtungen (insbesondere in einem Übergabebereich, in dem die Kunststoffvorformlinge von der ersten Sterilisationseinheit an die zweite Sterilisationseinheit übergeben werden) nicht miteinander kollidieren. Bevorzugt sind damit Elemente der Beaufschlagungseinrichtungen auf unterschiedlichen Höhen angeordnet.

Bei einer weiteren bevorzugten Ausführungsform sind auch die Beaufschlagungseinrichtungen und oder die Halteeinrichtungen der dritten Sterilisationseinheit auf einer anderen Höhe (in der Längsrichtung) der Kunststoffvorformlinge angeordnet wie die Beaufschlagungseinrichtungen und/oder Halteeinrichtungen der zweiten Sterilisationseinheit.

Bei einer weiteren bevorzugten Ausführungsform weisen die ersten Beaufschlagungseinrichtungen und/oder die zweiten Beaufschlagungseinrichtungen und/oder die dritten Beaufschlagungseinrichtungen Verteilelemente zum Verteilen des Sterilisationsmittel auf Mündungsbereiche der Kunststoffvorformlinge auf, wobei bevorzugt diese Verteilelemente einen größeren Querschnitt aufweisen als die Mündungsquerschnitte der zu sterilisierenden Behältnisse bzw. Kunststoffvorformlinge.

Auf diese Weise können die Kunststoffvorformlinge insbesondere im Bereich ihrer Mündung sowohl außen als auch innen sterilisiert werden. Besonders bevorzugt weisen diese Verteilelemente kreisförmige Querschnitte auf. Bei einer weiteren bevorzugten Ausführungsform sind diese Verteilelemente glockenartig (mit der Öffnung in Richtung der Kunststoffvorformlinge) ausgebildet.

Bei einer weiteren bevorzugten Ausführungsform weisen die Verteilelemente Umlenkelemente auf, um ein Sterilisationsmittel in das Innere der Kunststoffvorformlinge einzubringen.

Bei einer weiteren bevorzugten Ausführungsform sind die ersten Beaufschlagungseinrichtungen und oder die zweiten Beaufschlagungseinrichtungen bezüglich der zu Sterilisierenden Behältnisse in einer Längsrichtung der Behältnisse bewegbar. Auf diese Weise ist es möglich, dass die Beaufschlagungseinrichtungen beispielsweise näher an den Mündungen der Kunststoffvorformlinge geführt werden können, um so die Beaufschlagung mit dem Sterilisationsmittel effizienter zu gestalten. Dies kann dabei durch eine Bewegung der Beaufschlagungseinrichtungen in der Längsrichtung der Kunststoffvorformlinge erfolgen oder auch durch eine Bewegung der Kunststoffvorformlinge selbst. Daneben ist auch eine Kombination der beiden Bewegungen denkbar.

Bei einer weiteren bevorzugen Ausführungsform sind die ersten Halteeinrichtungen in einer ersten Position bezüglich einer Längsrichtung der Kunststoffvorformlinge angeordnet und die zweiten Halteeinrichtungen sind in einer zweiten Position bezüglich der Längsrichtung der Kunststoffvorformlinge angeordnet und die erste und die zweite Position sind unterschiedlich. So ist es beispielsweise möglich, dass die Kunststoffvorformlinge zunächst von den ersten Halteeinrichtungen unterhalb ihrs Tragrings gegriffen werden und dann von den zweiten Halteeinrichtungen oberhalb des Tragrings.

Auf diese Weise wird auch verhindert, dass es zu einer Kollision der Halteeinrichtungen kommt (in dem Übergabebereich der Kunststoffvorformlinge. Weiterhin sind bevorzugt auch die dritten Halteeinrichtungen (d. h. die Halteeinrichtungen der dritten Sterilisationseinheit) gegenüber den Halteeinrichtungen der zweiten Sterilisationseinheit auf einer anderen Höhenposition angeordnet. Auf diese Weise kann es auch zwischen den zweiten Halteeinrichtungen und den dritten Halteeinrichtungen nicht zu einer Kollision kommen.

Weiterhin weist bevorzugt die Sterilisationseinheit wenigstens eine Ventileinrichtung und bevorzugt eine Vielzahl von Ventileinrichtungen auf. Bevorzugt ist jeder Beaufschlagungseinrichtung wenigstens eine Ventileinrichtung zugeordnet, so dass die Zufuhr des fließfähigen Mediums an jeden einzelnen Kunststoffvorformling separat steuerbar ist. Diese Ventileinrichtungen können dabei in einem Bereich der Beaufschlagungseinrichtungen angeordnet sein oder aber auch in anderen Bereichen wie etwa in der Nähe des Drehverteilers.

Bei einer weiteren bevorzugten Ausführungsform weist die Sterilisationseinrichtung einen Reinraum auf, innerhalb dessen die Kunststoffvorformlinge transportiert werden. Dieser Reinraum grenzt dabei den Transportpfad der Kunststoffvorformlinge während deren Sterilisation gegenüber eine (unsterilen) Umgebung ab. Dabei ist es möglich, dass alle Sterilisationseinheiten innerhalb dieses Reinraums angeordnet sind. Bevorzugt ist jedoch die Erwärmungseinrichtung zum Erwärmen der Kunststoffvorformlinge außerhalb des Reinraums angeordnet.

Dieser Reinraum kann dabei zwei bezüglich einander bewegliche Wandungen aufweisen, welche den Reinraum begrenzen.

Bei einer weiteren bevorzugten Ausführungsform weist die Sterilisationseinrichtung wenigstens eine Teilungsverzugseinrichtung wie insbesondere einen Teilungsverzugsstern auf, der dazu geeignet und bestimmt ist, eine Teilung zwischen transportierten Kunststoffvorformlingen zu verändern und insbesondere zu erhöhen. Diese erste Teilungsverzugseinrichtung ist bevorzugt in der Transportrichtung der Kunststoffvorformlinge vor der ersten Sterilisationseinheit angeordnet.

Bei einer weiteren bevorzugten Ausführungsform weist die Vorrichtung eine zweite Teilungsverzugseinrichtung und bevorzugt einen zweiten Teilungsverzugsstern auf, der ebenfalls dazu geeignet und bestimmt ist, eine Teilung der Kunststoffvorformlinge zu verändern und insbesondere zu erhöhen. Bevorzugt ist dieser zweite Teilungsverzugsstern in der Transportrichtung der Kunststoffvorformlinge nach der letzten, beispielsweise nach der dritten Sterilisationseinheit angeordnet.

Die vorliegende Erfindung ist weiterhin auf ein Verfahren zum Sterilisieren von Kunststoffbehältnissen und insbesondere von Kunststoffvorformlingen gerichtet, wobei die Kunststoffvorformlinge mittels einer ersten Sterilisationseinrichtung sterilisiert werden, welche wenigstens eine erste Sterilisationseinheit aufweist, welche einen drehbaren Transportträger und eine Vielzahl von an diesem Transportträger angeordnete erste Halteeinrichtungen, welche die Kunststoffvorformlinge halten, aufweist sowie eine Vielzahl von ersten Beaufschlagungseinrichtungen, welche die Kunststoffvorformlinge mit einem fließfähigem Medium beaufschlagen.

Weiterhin weist die Sterilisationseinrichtung eine zweite Sterilisationseinheit auf, welche die Kunststoffvorformlinge nach deren Sterilisation durch die erste Sterilisationseinheit sterilisiert und welche einen (zweiten) drehbaren Transportträger und eine Vielzahl von an diesem Transportträger angeordnete zweite Halteeinrichtungen, welche die Kunststoffvorformlinge halten sowie eine Vielzahl von zweiten Beaufschlagungseinrichtungen welche die Kunststoffvorformlinge mit einem fließfähigem Medium beaufschlagen.

Erfindungsgemäß werden die Kunststoffvorformlinge unmittelbar von der ersten Sterilisationseinheit an die zweite Sterilisationseinheit übergeben.

Es wird daher auch verfahrensseitig vorgeschlagen, dass die Kunststoffvorformlinge unmittelbar von der ersten Sterilisationseinheit bzw. von einer Halteeinrichtung der ersten Sterilisationseinheit an eine Halteeinrichtung der zweiten Sterilisationseinheit übergeben werden.

Bei einem bevorzugten Verfahren werden zunächst die Kunststoffvorformlinge in einer Erwärmungseinrichtung erwärmt. Anschließend wird bevorzugt eine Teilung der Kunststoffvorformlinge verändert und insbesondere erhöht.

Anschließend werden die Kunststoffvorformlinge mittels der ersten Sterilisationseinheit sterilisiert und insbesondere mit H₂O₂ Prozessgas behandelt bzw. beaufschlagt. Dabei können die Kunststoffvorformlinge beispielsweise unterhalb ihres Tragrings gegriffen werden. In einem weiteren Schritt werden die Kunststoffvorformlinge mit der zweiten Sterilisationseinheit behandelt, was bevorzugt ebenfalls mit H₂O₂ Prozessgas erfolgt. Dabei werden bevorzugt die Kunststoffvorformlinge oberhalb ihres Tragrings gegriffen.

Anschließend werden bevorzugt die Kunststoffvorformlinge mit der dritten Sterilisationseinheit behandelt, wobei auch hier bevorzugt wieder die Behandlung mit H₂O₂ Prozessgas erfolgt. Dabei werden bevorzugt die Kunststoffvorformlinge wieder unterhalb ihres Tragrings gegriffen.

Anschließend wird optional wieder eine Teilung der Kunststoffvorformlinge mittels einer zweiten Teilungsverzugseinrichtung weiter verändert und insbesondere weiter erhöht, insbesondere um die Kunststoffvorformlinge in einem weiteren Schritt an eine Umformungseinrichtung wie insbesondere eine Streckblasmaschine zu übergeben.

Weitere Vorteile und Ausführungsformen ergeben sich aus den beigefügten Zeichnungen: Darin zeigen:
- Fig. 1: eine schematische Darstellung einer Vorrichtung zum Behandeln von Behältnissen;
- Fig. 2: eine schematische Darstellung einer Sterilisationseinheit;
- Fig. 3: eine Darstellung einer Beaufschlagungseinrichtung;
- Fig. 4: eine Schnittdarstellung zweier Beaufschlagungseinrichtungen in einem Übergabebereich;
- Fig. 5: eine weitere Darstellung der Übergabe von einem Kunststoffvorformling von einer Sterilisationseinheit an eine andere Sterilisationseinheit.

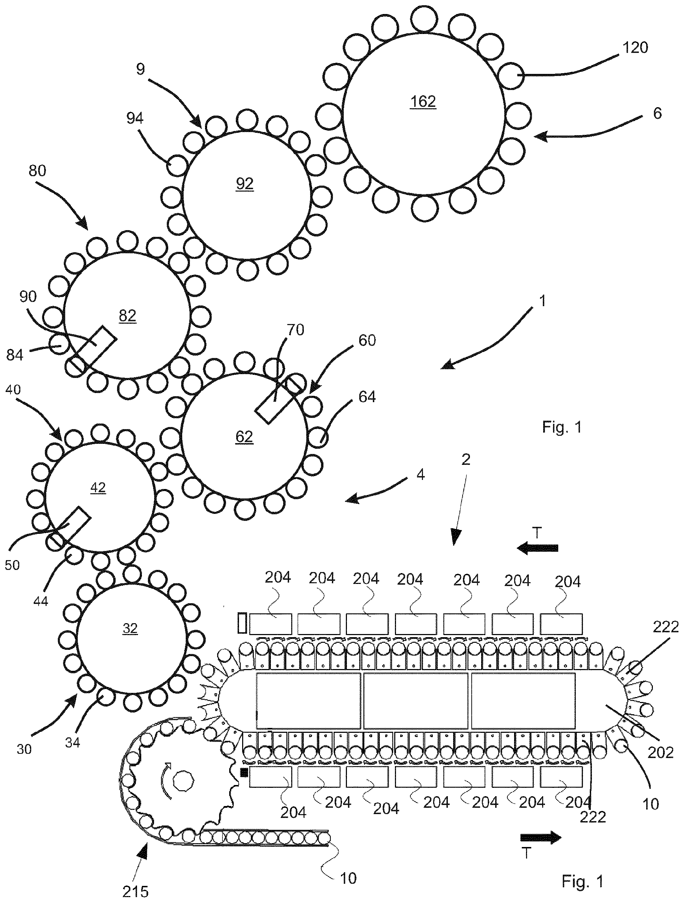

Figur 1 zeigt eine Darstellung einer Vorrichtung 1 zum Herstellen von Behältnissen. Dabei werden zunächst Kunststoffvorformlinge 10 mittels einer Zufuhreinrichtung 215 wie eines Eintaktsterns einem in seiner Gesamtheit mit 2 bezeichneten Ofen zugeführt. Dieser Ofen 2 weist eine Vielzahl von Heizeinrichtungen 204 auf, die stationär entlang des Transportpfads der Kunststoffvorformlinge angeordnet sind. Die Kunststoffvorformlinge 10 werden an diesen Heizeinrichtungen 204 mittels einer Transporteinrichtung 202 und Halteelementen 222 vorbeigeführt und dabei erwärmt.

An diese Erwärmungseinrichtung 2 schließt sich eine erste Transporteinrichtung in Form eines Teilungsverzugssterns 30 an. Dieser erhöht die Teilung zwischen den einzelnen Kunststoffvorformlingen, d. h. deren Abstand zueinander. Dieser Teilungsverzugsstern weist einen drehbaren Träger auf, an dem eine Vielzahl von Halteeinrichtungen 34 zum Halten der Kunststoffvorformlinge angeordnet ist. Diese sind dabei bevorzugt derart beweglich, dass die Teilung zwischen den Kunststoffvorformlingen 10 verändert und insbesondere vergrößert bzw. erhöht wird. Dieser Teilungsverzugsstern ist dabei bevorzugt bereits innerhalb eines nicht dargestellten Reinraums angeordnet und ist bevorzugt auch Bestandteil des Sterilisationsmoduls. Bevorzugt werden die Kunststoffvorformlinge von dem Teilungsverzugsstern im Uhrzeigersinn transportiert.

An diese erste Transporteinrichtung schließt sich eine in ihrer Gesamtheit mit 4 bezeichnete Sterilisationseinrichtung an. Diese Sterilisationseinrichtung weist dabei eine erste Sterilisationseinheit 40 auf. Diese erste Sterilisationseinheit 40 weist einen drehbaren Träger 42 auf, an dem eine Vielzahl von Halteeinrichtungen 44 zum Halten der Kunststoffvorformlinge angeordnet ist. Zusätzlich ist eine Vielzahl von Beaufschlagungseinrichtungen (in ihrer Gesamtheit mit 50 bezeichnet) vorgesehen, welche die Kunststoffvorformlinge mit einem Sterilisationsmedium wie beispielsweise Wasserstoffperoxid beaufschlagen. Bevorzugt werden die Kunststoffvorformlinge von der ersten Sterilisationseinheit 40 entgegen des Uhrzeigersinns transportiert.

An die erste Sterilisationseinheit 40 schließt sich eine zweite Sterilisationseinheit 60 an. Diese zweite Sterilisationseinheit 60 weist ebenfalls einen drehbaren Transportträger 62 auf, sowie eine Vielzahl von Halteeinrichtungen 64, welche ebenfalls zum Halten der Kunststoffvorformlingen dienen. Daneben weist diese zweite Sterilisationseinheit 60 eine Vielzahl von Beaufschlagungseinrichtungen 70 (nur eine gezeigt) auf, welche die Kunststoffvorformlinge ebenfalls mit einem (insbesondere fließfähigem) Sterilisationsmedium beaufschlagen. Bevorzugt werden die Kunststoffvorformlinge von der zweitem Sterilisationseinheit 60 im Uhrzeigersinn transportiert.

An die zweite Sterilisationseinheit 60 schließt sich eine dritte Sterilisationseinheit 80 an. Diese weist ebenfalls einen Transportträger 82 auf, so wie eine Vielzahl von Halteeinrichtungen 84, die an diesen Transportträger 82 angeordnet sind. Daneben ist auch hier wieder eine Vielzahl von Beaufschlagungseinrichtungen 90 (nur eine gezeigt) vorgesehen, welche die Kunststoffvorformlinge ebenfalls mit einem Sterilisationsmedium beaufschlagen. Bevorzugt werden die Kunststoffvorformlinge von der dritten Sterilisationseinheit 80 entgegen des Uhrzeigersinns transportiert.

An die dritte Sterilisationseinheit 80 schließt sich bevorzugt eine weitere Transporteinrichtung 90 in Form eines zweiten Teilungsverzugssterns 90 an. Dieser zweite Teilungsverzugsstern weist ebenfalls einen drehbaren Träger 92 auf, an dem eine Vielzahl von Halteeinrichtungen 94 zum Halten der Kunststoffvorformlinge angeordnet sind. Dieser zweite Teilungsverzugsstern erhöht ebenfalls die Teilung zwischen den Kunststoffvorformlingen. Der zweite Teilungsverzugsstern 90 kann dabei ebenfalls noch Bestandteil der Sterilisationseinrichtung 4 sein und ebenfalls noch in dem (nicht gezeigten) Reinraum angeordnet sein. Bevorzugt werden die Kunststoffvorformlinge von dem zweiten Teilungsverzugsstern 90 im Uhrzeigersinn transportiert.

An die Sterilisationseinrichtung 4 schließt sich eine Umformungseinrichtung 6, wie insbesondere eine Streckblasmaschine an. Diese weist ebenfalls einen Transportträger 162 auf, sowie auch eine Vielzahl von Umformungsstationen 120, welche jeweils die Kunststoffvorformlinge - hier durch Beaufschlagung mit Druckluft - zu den Kunststoffbehältnissen umformen.

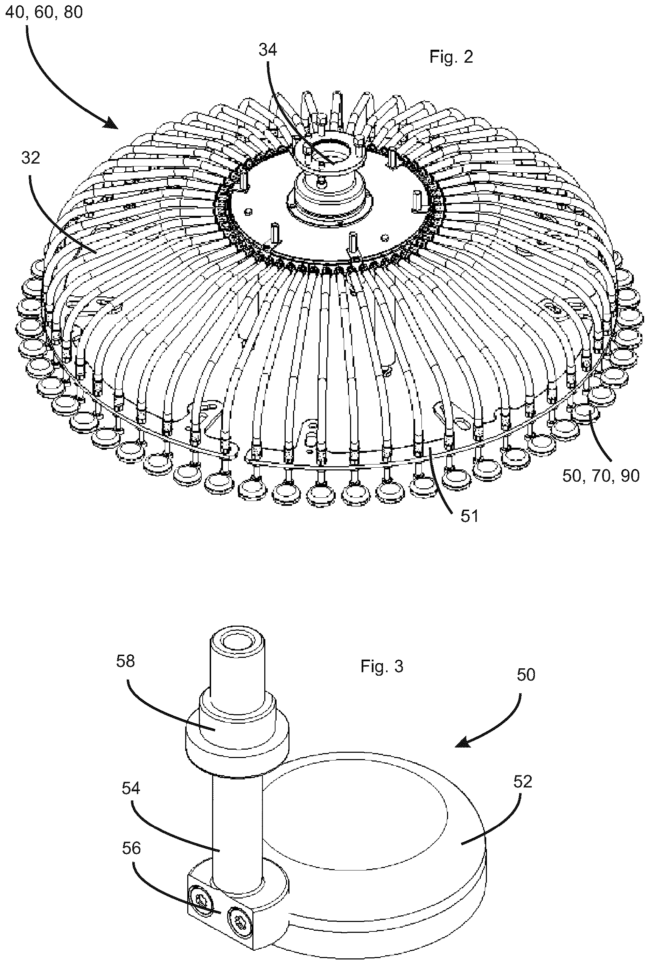

Figur 2 zeigt eine Darstellung einer Sterilisationseinheit 40, 60, 80. Diese Sterilisationseinheiten können dabei im Wesentlichen gleichartig ausgebildet sein. Die Sterilisationseinheit 40, 60, 80 weist dabei eine Vielzahl von Beaufschlagungseinrichtungen 50,70, 90 auf. Diesen wird jeweils über Zuführleitungen 32 das Sterilisationsmittel zugeführt. Das Bezugszeichen 34 kennzeichnet eine Verteileinrichtung wie einen Drehverteiler, welcher das Sterilisationsmedium auf die einzelnen Beaufschlagungseinrichtungen 50,70, 90 verteilt.

Das Bezugszeichen 51 kennzeichnet einen nur teilweise dargestellten Transportstern, an dem die einzelnen Beaufschlagungseinrichtungen befestigt sind. Dabei ist es möglich, dass auch dieser Transportstern wiederum in den in Figur 1 gezeigten Träger 42, 62, 82 gefestigt ist, es wäre jedoch auch möglich, dass ein weiterer separater insbesondere scheibenförmiger Träger vorgesehen ist, der jedoch in Figur 2 nicht dargestellt ist.

Figur 3 zeugt eine Beaufschlagungseinheit 50. Diese weist eine Zuführleitung 54 auf, die in einem Anschluss 54 auf, die an einem Anschluss 58 angeordnet ist und welche das Sterilisationsmedium über eine Umlenkung 56 einer Verteileinrichtung 52 zuführt. Diese Verteileinrichtung führt das Sterilisationsmedium zu dem Mündungsbereich der Kunststoffvorformlinge.

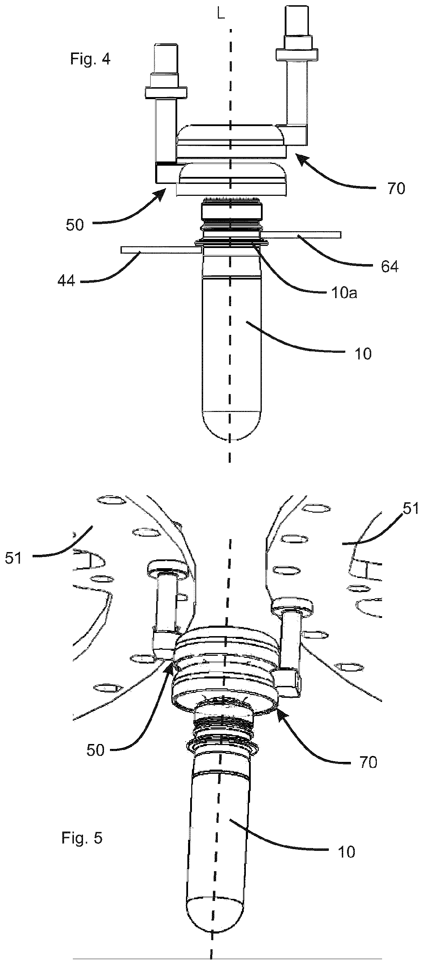

Figur 4 zeigt eine Darstellung eines Kunststoffvorformlings 10, der in einem Übergabebereich geführt wird, in dem er von einer Sterilisationseinheit beispielsweise der ersten Sterilisationseinheit 40 an eine andere Sterilisationseinheit übergeben wird. Dabei bezieht sich das Bezugszeichen L auf die Längsrichtung des Kunststoffvorformlings. Man erkennt, dass in dieser Längsrichtung L die zwei Beaufschlagungseinrichtungen 50 und 70 versetzt übereinander angeordnet sind, so dass sie nicht miteinander kollidieren können. Auch die Halteeinrichtungen 44 und 64, die den beiden Sterilisationseinheiten zugeordnet sind, sind auf unterschiedlicher Höhe (versetzt angeordnet), so dass sie den Kunststoffvorformling 10 einerseits unterhalb dessen Tragring 10a und andererseits oberhalb dessen Tragring greifen können. Auf diese Weise kann es auch zu keiner Kollusion der Halteeinrichtungen kommen.

Daneben ist es möglich, dass im Betrieb die Beaufschlagungseinrichtungen 50 oder 70 in der Längsrichtung L verschiebbar sind, insbesondere jedoch in einem Bereich außerhalb des gezeigten Übergabebereichs. Dabei ist zu berücksichtigen, dass außerhalb des Übergabebereichs lediglich eine der beiden Beaufschlagungseinrichtungen und lediglich auch eine der beiden Halteeinrichtungen vorgesehen ist. Zum Zwecke des Heranführens der Beaufschlagungseinrichtung an den Kunststoffvorformling kann beispielsweise der Kunststoffvorformling 10 angehoben werden oder auch umgekehrt die Beaufschlagungseinrichtung 50 oder 70 abgesenkt werden.

Figur 5 zeigt eine weitere Veranschaulichung der Übergabe eines Kunststoffvorformlings von einer Sterilisationseinheit an eine weitere Sterilisationseinheit. Dabei sind wieder die beiden Träger 51 dargestellt an denen jeweils die Beaufschlagungseinrichtungen 50,70 angeordnet sind. Man erkennt, dass in diesem Übergabebereich nicht zu einer Kollision zwischen den Beaufschlagungseinrichtungen kommt.

## Patentansprüche

1. Sterilisationseinrichtung (4) zum Sterilisieren von Kunststoffbehältnissen und insbesondere von Kunststoffvorformlingen (10), wobei die Sterilisationseinrichtung (4) wenigstens eine erste Sterilisationseinheit (40) aufweist, welche einen drehbaren Transportträger (42) und eine Vielzahl von an diesem Transportträger (42) angeordneten ersten Halteeinrichtungen (44) zum Halten der Kunststoffvorformlinge sowie eine Vielzahl von ersten Beaufschlagungseinrichtungen (50) zum Beaufschlagen der Kunststoffvorformlinge (10) mit einem fließfähigen Sterilisationsmedium aufweist und die Sterilisationseinrichtung eine zweite Sterilisationseinrichtung (60) aufweist, welche sich in der Transportrichtung der Kunststoffvorformlinge an die erste Sterilisationseinrichtung (40) anschließt und welche einen drehbaren Transportträger (62) und eine Vielzahl von an diesem Transportträger (62) angeordneten zweiten Halteeinrichtungen (64) zum Halten der Kunststoffvorformlinge (10) aufweist sowie eine Vielzahl von zweiten Beaufschlagungseinrichtungen (70) zum Beaufschlagen der Kunststoffvorformlinge (10) mit einem fließfähigen Sterilisationsmedium, und die Kunststoffvorformlinge unmittelbar von der ersten Sterilisationseinheit (40) an die zweite Sterilisationseinheit (60) übergebbar sind,
**dadurch gekennzeichnet, dass**
die ersten Beaufschlagungseinrichtungen (50) oder Bestandteile der ersten Beaufschlagungseinrichtungen (70) in einer ersten Position (P1) bezüglich einer Längsrichtung (L) der Kunststoffvorformlinge angeordnet sind und die zweiten Beaufschlagungseinrichtungen (70) oder Bestanteile der zweiten Beaufschlagungseinrichtungen (70) in einer zweiten Position (P2) bezüglich der Längsrichtung (L) der Kunststoffvorformlinge angeordnet sind und die ersten und zweiten Positionen unterschiedlich sind.

2. Sterilisationseinrichtung (4) nach dem vorangegangenen Anspruch,
**dadurch gekennzeichnet, dass**
jeder ersten Halteeinrichtung (44) genau eine erste Beaufschlagungseinrichtung (50) zugeordnet ist und/oder jeder zweiten Halteeinrichtung (64) genau eine zweite Beaufschlagungseinrichtung (70) zugeordnet ist.

3. Sterilisationseinrichtung nach wenigstens einem der vorangegangenen Ansprüche 1 - 2,
**dadurch gekennzeichnet, dass**
die ersten Beaufschlagungseinrichtungen (50) und/oder die zweiten Beaufschlagungseinrichtungen (70) an einem drehbaren Träger (42, 62) angeordnet sind.

4. Sterilisationseinrichtung nach wenigstens einem der vorangegangenen Ansprüche 1 -3,
**dadurch gekennzeichnet, dass**
die Sterilisationseinrichtung (4) eine dritte Sterilisationseinheit (80) aufweist, welche einen drehbaren Transportträger (82) und eine Vielzahl von an diesem Transportträger (82) angeordnete Halteeinrichtungen (84) zum Halten der Kunststoffvorformlinge sowie eine Vielzahl von Beaufschlagungseinrichtungen (90) zum Beaufschlagen der Kunststoffvorformlinge (10) mit einem fließfähigen Sterilisationsmedium aufweist.

5. Sterilisationseinrichtung nach wenigstens einem der vorangegangenen Ansprüche 1 -4,
**dadurch gekennzeichnet, dass**
die ersten Beaufschlagungseinrichtungen (50) und/oder die zweiten Beaufschlagungseinrichtungen (70) Verteilelemente (52, 72) zum Verteilen eines Sterilisationsmittels auf Mündungsbereiche der Behältnisse aufweisen, wobei diese Verteilelemente einen größeren Querschnitt aufweisen, als die Mündungsquerschnitte der zu sterilisierenden Behältnisse.

6. Sterilisationseinrichtung nach wenigstens einem der vorangegangenen Ansprüche 1 -5,
**dadurch gekennzeichnet, dass**
die ersten Beaufschlagungseinrichtungen (60) und/oder die zweiten Beaufschlagungseinrichtungen (70) bezüglich der zu sterilisierenden Behältnisse in einer Längsrichtung der Behältnisse bewegbar sind.

7. Sterilisationseinrichtung nach wenigstens einem der vorangegangenen Ansprüche 1 -6,
**dadurch gekennzeichnet, dass**
die ersten Halteeinrichtungen (44) in einer ersten Position (P11) bezüglich einer Längsrichtung (L) der Kunststoffvorformlinge angeordnet sind und die zweiten Halteeinrichtungen in einer zweiten Position (P12) bezüglich der Längsrichtung (L) der Kunststoffvorformlinge angeordnet sind und die ersten und zweiten Positionen unterschiedlich sind.

8. Vorrichtung zum Behandeln von Kunststoffvorformlingen (10) mit einer Erwärmungseinrichtung (2) zum Erwärmen der Kunststoffvorformlinge, wobei die Erwärmungseinrichtung (2) eine Transporteinrichtung (202) zum Transportieren der Kunststoffvorformlinge (10) aufweist sowie wenigstens eine Heizeinrichtung (204) zum Erwärmen der Kunststoffvorformlinge (10), mit einer in einer Transportrichtung der Kunststoffvorformlinge (10) der Erwärmungseinrichtung (2) nachgeordneten Umformungseinrichtung (6) zum Umformen der Kunststoffvorformlinge (10) zu Kunststoffbehältnissen und mit einer Sterilisationseinrichtung (4) zum Sterilisieren der Kunststoffvorformlinge (10) nach wenigstens einem der Ansprüche 1-7.

9. Vorrichtung (1) nach Anspruch 8,
**dadurch gekennzeichnet, dass**
die Kunststoffvorformlinge (10) unmittelbar von einer Halteeinrichtung (42) der ersten Sterilisationseinheit (40) an eine Halteeinrichtung (62) der zweiten Sterilisationseinheit (60) übergebbar sind.

10. Vorrichtung (1) nach wenigstens einem der vorangegangenen Ansprüche 8-9,
**dadurch gekennzeichnet, dass**
die Sterilisationseinrichtung in der Transportrichtung der Kunststoffvorformlinge zwischen der Erwärmungseinrichtung (2) und der Umformungseinrichtung (6) angeordnet ist.

11. Verfahren zum Sterilisieren von Kunststoffbehältnissen und insbesondere von Kunststoffvorformlingen (10), wobei die Kunststoffbehältnisse mittels einer ersten Sterilisationseinrichtung (4) sterilisiert werden, welche wenigstens eine erste Sterilisationseinheit (40) aufweist, welche einen drehbaren Transportträger (42) und eine Vielzahl von an diesem Transportträger (42) angeordnete erste Halteeinrichtungen (44), welche die Kunststoffvorformlinge halten aufweist, sowie eine Vielzahl von ersten Beaufschlagungseinrichtungen (50), welche die Kunststoffvorformlinge (10) mit einem fließfähigen Medium beaufschlagen und die Sterilisationseinrichtung eine zweite Sterilisationseinheit (60) aufweist, welche die Kunststoffvorformlinge nach deren Sterilisation durch die erste Sterilisationseinheit (40) sterilisiert und welche einen drehbaren Transportträger (62) und eine Vielzahl von an diesem Transportträger (62) angeordnete zweite Halteeinrichtungen (64) welche die Kunststoffvorformlinge (10) halten sowie eine Vielzahl von zweiten Beaufschlagungseinrichtungen (70) welche die Kunststoffvorformlinge (10) mit einem fließfähigen Medium beaufschlagen, und die Kunststoffvorformlinge unmittelbar von der ersten Sterilisationseinheit (40) an die zweite Sterilisationseinheit (60) übergeben werden,
**dadurch gekennzeichnet, dass**
die ersten Beaufschlagungseinrichtungen (50) oder Bestandteile der ersten Beaufschlagungseinrichtungen (70) in einer ersten Position (P1) bezüglich einer Längsrichtung (L) der Kunststoffvorformlinge angeordnet sind und die zweiten Beaufschlagungseinrichtungen (70) oder Bestanteile der zweiten Beaufschlagungseinrichtungen (70) in einer zweiten Position (P2) bezüglich der Längsrichtung (L) der Kunststoffvorformlinge angeordnet sind und die ersten und zweiten Positionen unterschiedlich sind.

## Claims

1. Sterilization device (4) for sterilizing plastic containers and in particular plastic preforms (10), wherein the sterilization device (4) having at least one first sterilization unit (40) which has a rotatable transport carrier (42) and a plurality of first holding devices (44), arranged on this transport carrier (42), for holding the plastic preforms, and a plurality of first application devices (50) for acting upon the plastic preforms (10) with a flowable sterilization medium, and the sterilization device has a second sterilization device (60) which follows the first sterilization device (40) in the transport direction of the plastic preforms and which has a rotatable transport carrier (62) and a plurality of second holding devices (64), arranged on this transport carrier (62), for holding the plastic preforms (10), and a plurality of second application devices (70) for acting upon the plastic preforms (10) with a flowable sterilization medium, and the plastic preforms can be transferred directly from the first sterilization unit (40) to the second sterilization unit (60),
**characterized in that**
the first application devices (50) or components of the first application devices (70) are arranged in a first position (P1) with respect to a longitudinal direction (L) of the plastic preforms and the second application devices (70) or components of the second application devices (70) are arranged in a second position (P2) with respect to a longitudinal direction (L) of the plastic preforms and the first and the second positions are different.

2. Sterilization device (4) according to the preceding claim,
**characterized in that**
precisely one first application device (50) is assigned to each first holding device (44) and/or precisely one second application device (70) is assigned to each second holding device (64).

3. Sterilization device according to at least one of the preceding claims 1 - 2, **characterized in that**
the first application devices (50) and/or the second application devices (70) are arranged on a rotatable carrier (42, 62).

4. Sterilization device according to at least one of the preceding claims 1 - 3, **characterized in that**
the sterilization device (4) comprises a third sterilization unit (80), which comprises a rotatable transport carrier (82) and a plurality of holding devices (84) arranged on this transport carrier (82) for holding the plastic preforms, as well as a plurality of application devices (90) for acting upon the plastic preforms (10) with a flowable sterilization medium.

5. Sterilization device according to at least one of the preceding claims 1 - 4, **characterized in that**
the first application devices (50) and/or the second application devices (70) have distributing elements (52, 72) for distributing a sterilizing agent to mouth regions of the containers, wherein these distributing elements having a larger cross section than the mouth cross sections of the containers to be sterilized.

6. Sterilization device according to at least one of the preceding claims 1 - 5, **characterized in that**
the first application devices (60) and/or the second application devices (70) are movable with respect to the containers to be sterilized in a longitudinal direction of the containers.

7. Sterilization device according to at least one of the preceding claims 1 - 6, **characterized in that**
the first holding means (44) are arranged in a first position (P11) with respect to a longitudinal direction (L) of the plastic preforms and the second holding means are arranged in a second position (P12) with respect to the longitudinal direction (L) of the plastic preforms, and the first and second positions are different.

8. Apparatus for treating plastic preforms (10) with a heating device (2) for heating the plastic preforms, wherein the heating device (2) has a transport device (202) for transporting the plastic preforms (10) and at least one heating device (204) for heating the plastic preforms (10), having a forming device (6), arranged downstream of the heating device (2) in a transport direction of the plastic preforms (10), for forming the plastic preforms (10) into plastic containers, and having a sterilization device (4) for sterilizing the plastic preforms (10) according to at least one of claims 1 -7.

9. Apparatus (1) according to claim 8,
**characterized in that**
the plastic preforms (10) are transferred directly from a holding device (42) of the first sterilization unit (40) to a holding device (62) of the second sterilization unit (60).

10. Apparatus (1) according to at least one of the preceding claims 8-9, **characterized in that**
the sterilization device is arranged in the transport direction of the plastic preforms between the heating device (2) and the forming device (6).

11. Method for sterilizing plastic containers and in particular plastic preforms (10), wherein the plastic containers are sterilized by means of a first sterilization device (4) which comprises at least a first sterilization unit (40) which has a rotatable transport carrier (42) and a plurality of first holding devices (44) arranged on said transport carrier (42) which hold the plastic preforms, and a plurality of first application devices (50) which act upon the plastic preforms (10) with a flowable medium, and the sterilization device has a second sterilization unit (60) which sterilizes the plastic preforms after they have been sterilized by the first sterilization unit (40) and which has a rotatable transport carrier (62) and a plurality of second holding devices (64) which are arranged on this transport carrier (62) and hold the plastic preforms (10), and a plurality of second application devices (70) which act upon the plastic preforms (10) with a flowable medium, and the plastic preforms are transferred directly from the first sterilization unit (40) to the second sterilization unit (60),
**characterized in that**
the first application devices (50) or components of the first application devices (70) are arranged in a first position (P1) with respect to a longitudinal direction (L) of the plastic preforms and the second application devices (70) or components of the second application devices (70) are arranged in a second position (P2) with respect to a longitudinal direction (L) of the plastic preforms and the first and the second positions are different.

## Revendications

1. Système de stérilisation (4) destiné à stériliser des récipients en matière plastique et en particulier des préformes en matière plastique (10), dans lequel le système de stérilisation (4) présente au moins une première unité de stérilisation (40), laquelle présente un support de transport (42) rotatif et une pluralité de premiers systèmes de maintien (44) disposés sur ledit support de transport (42), destinés à maintenir les préformes en matière plastique ainsi qu'une pluralité de premiers systèmes de sollicitation (50) destinés à soumettre les préformes en matière plastique (10) à l'action d'un milieu de stérilisation pouvant s'écouler et le système de stérilisation présente un deuxième système de stérilisation (60), lequel se raccorde au premier système de stérilisation (40) dans la direction de transport des préformes en matière plastique et lequel présente un support de transport (62) rotatif et une pluralité de deuxièmes systèmes de maintien (64) disposés sur ledit support de transport (62), destinés à maintenir les préformes en matière plastique (10) ainsi qu'une pluralité de deuxièmes systèmes de sollicitation (70) destinés à soumettre les préformes en matière plastique (10) à l'action d'un milieu de stérilisation pouvant s'écouler, et les préformes en matière plastique peuvent être transférées directement depuis la première unité de stérilisation (40) à la deuxième unité de stérilisation (60),
**caractérisé en ce que**
les premiers systèmes de sollicitation (50) ou des parties intégrantes des premiers systèmes de sollicitation (70) sont disposés dans une première position (P1) par rapport à une direction longitudinale (L) des préformes en matière plastique, et les deuxièmes systèmes de sollicitation (70) ou des parties intégrantes des deuxièmes systèmes de sollicitation (70) sont disposés dans une deuxième position (P2) par rapport à la direction longitudinale (L) des préformes en matière plastique et les premières et deuxièmes positions sont différentes.

2. Système de stérilisation (4) selon la revendication précédente, **caractérisé en ce que**
précisément un premier système de sollicitation (50) est associé à chaque premier système de maintien (44) et/ou précisément un deuxième système de sollicitation (70) est associé à chaque deuxième système de maintien (64).

3. Système de stérilisation selon au moins l'une quelconque des revendications précédentes 1 - 2,
**caractérisé en ce que**
les premiers systèmes de sollicitation (50) et/ou les deuxièmes systèmes de sollicitation (70) sont disposés sur un support (42, 62) rotatif.

4. Système de stérilisation selon au moins l'une quelconque des revendications précédentes 1 - 3,
**caractérisé en ce que**
le système de stérilisation (4) présente une troisième unité de stérilisation (80), laquelle présente un support de transport (82) rotatif et une pluralité de systèmes de maintien (84) disposés sur ledit support de transport (82), destinés à maintenir les préformes en matière plastique ainsi qu'une pluralité de systèmes de sollicitation (90) destinés à soumettre les préformes en matière plastique (10) à l'action d'un milieu de stérilisation pouvant s'écouler.

5. Système de stérilisation selon au moins l'une quelconque des revendications précédentes 1 - 4,
**caractérisé en ce que**
les premiers systèmes de sollicitation (50) et/ou les deuxièmes systèmes de sollicitation (70) présentent des éléments de répartition (52, 72) destinés à répartir un produit de stérilisation sur des zones d'embouchure des récipients, dans lequel lesdits éléments de répartition présentent une section transversale plus grande que les sections transversales d'embouchure des récipients à stériliser.

6. Système de stérilisation selon au moins l'une quelconque des revendications précédentes 1 - 5,
**caractérisé en ce que**
les premiers systèmes de sollicitation (60) et/ou les deuxièmes systèmes de sollicitation (70) peuvent être déplacés par rapport aux récipients à stériliser dans une direction longitudinale des récipients.

7. Système de stérilisation selon au moins l'une quelconque des revendications précédentes 1 - 6,
**caractérisé en ce que**
les premiers systèmes de maintien (44) sont disposés dans une première position (P11) par rapport à une direction longitudinale (L) des préformes en matière plastique et les deuxièmes systèmes de maintien sont disposés dans une deuxième position (P12) par rapport à la direction longitudinale (L) des préformes en matière plastique et les premières et deuxièmes positions sont différentes.

8. Dispositif de traitement de préformes en matière plastique (10) avec un système de chauffage (2) destiné à chauffer les préformes en matière plastique, dans lequel le système de chauffage (2) présente un système de transport (202) destiné à transporter les préformes en matière plastique (10), ainsi qu'au moins un système chauffant (204) destiné à chauffer les préformes en matière plastique (10) avec un système de façonnage (6) disposé en aval du système de chauffage (2) dans une direction de transport des préformes en matière plastique (10), destiné à façonner les préformes en matière plastique (10) en des récipients en matière plastique et avec un système de stérilisation (4) destiné à stériliser les préformes en matière plastique (10) selon au moins l'une quelconque des revendications 1 - 7.

9. Dispositif (1) selon la revendication 8,
**caractérisé en ce que**
les préformes en matière plastique (10) peuvent être transférées directement d'un système de maintien (42) de la première unité de stérilisation (40) à un système de maintien (62) de la deuxième unité de stérilisation (60).

10. Dispositif (1) selon au moins l'une quelconque des revendications précédentes 8 - 9,
**caractérisé en ce que**
le système de stérilisation est disposé dans la direction de transport des préformes en matière plastique entre le système de chauffage (2) et le système de façonnage (6).

11. Procédé de stérilisation de récipients en matière plastique et en particulier de préformes en matière plastique (10), dans lequel les récipients en matière plastique sont stérilisés au moyen d'un premier système de stérilisation (4), lequel présente au moins une première unité de stérilisation (40), laquelle présente un support de transport (42) rotatif et une pluralité de premiers systèmes de maintien (44) disposés sur ledit support de transport (42), lesquels maintiennent les préformes en matière plastique, ainsi qu'une pluralité de premiers systèmes de sollicitation (50), lesquelles soumettent les préformes en matière plastique (10) à l'action d'un milieu pouvant s'écouler, et le système de stérilisation présente une deuxième unité de stérilisation (60), laquelle stérilise les préformes en matière plastique après leur stérilisation par la première unité de stérilisation (40) et laquelle présente un support de transport (62) rotatif et une pluralité de deuxièmes systèmes de maintien (64) disposés sur ledit support de transport (62), lesquels maintiennent les préformes en matière plastique (10), ainsi qu'une pluralité de deuxièmes systèmes de sollicitation (70), lesquels sollicitent les préformes en matière plastique (10) à l'action d'un milieu pouvant s'écouler, et les préformes en matière plastique sont transférées directement depuis la première unité de stérilisation (40) à la deuxième unité de stérilisation (60),
**caractérisé en ce que**
les premiers systèmes de sollicitation (50) ou des parties intégrantes des premiers systèmes de sollicitation (70) sont disposés dans une première position (P1) par rapport à une direction longitudinale (L) des préformes en matière plastique et les deuxièmes systèmes de sollicitation (70) ou parties intégrantes des deuxièmes systèmes de sollicitation (70) sont disposés dans une deuxième position (P2) par rapport à la direction longitudinale (L) des préformes en matière plastique et les premières et deuxièmes positions sont différentes.
